# EUROPEAN PATENT APPLICATION

(11) **EP 2 455 748 A1**
(43) Date of publication of application: **23.05.2012**
(21) Application number: 10014711.5
(22) Date of filing: 17.11.2010
(51) Int. Cl.: G01N 27/30, C12Q 1/00

(54) **Sensor device, in particular for substance sensing, measuring apparatus, and method of manufacturing the sensor device**

(71) Applicant: Max-Planck-Gesellschaft zur Förderung der Wissenschaften e.V., 80539 München (DE); Queen Mary and Westfield College, London E1 4NS (GB)
(72) Inventor: Russel, Philip, 91341 Röttenbach (DE); Schmidt, Markus, W 3 7ST, London (GB); Kacanovska, Anna, Isleworth, TW7 6ET (GB); Vadgama, Pankaj, Loughton, Essex, IG10 1NX (GB)
(74) Representative: Hertz, Oliver

(57) **Abstract**

A sensor device 100, in particular being adapted for sensing of substances, which comprises a sensor filament 10, which has a first end providing an exposed sensing surface 11 and a second end 12 being connectable with a metering device 220, and a recessed sensor tip 20, which has an inner space 21 to which the sensing surface 11 is exposed, further including a first capillary 13 including the sensor filament 10 and having a first capillary end 14, where the sensing surface 11 is exposed to the inner space 21 of the recessed sensor tip 20, and a second capillary 22 being fixedly connected to the first capillary end 14 and providing the recessed sensor tip 20. Furthermore, a measuring apparatus 200 including the sensor device 100 and a method of manufacturing the sensor device 100 are described.

## Description

### Field of the invention

The present invention relates to a sensor device, in particular being adapted for sensing of substances, e. g. for electrochemical or optical sensing of substances, like a sensor for bio-molecules. Furthermore, the invention relates to a measuring apparatus including the sensor device. Furthermore, the invention relates to a method of manufacturing the sensor device. Applications of the invention are present in the field of sensing chemical substances, in particular in the field of electrochemical sensing.

### Background of the invention

Electrochemical sensing of substances is generally known and broadly applied, in particular since the development of the so called Clark electrode for sensing an oxygen concentration in a solution. A sensor electrode and a reference electrode connected via an amp meter are immersed into a solution. The molecule, e. g. oxygen or glucose to be sensed is subjected to a first electrochemical reaction at the sensor electrode, while another electrochemical reaction is induced at the reference electrode so that an electrical current is flowing via the amp meter. The electrical current is a direct quantitative measure of the molecule to be sensed. With the Clark electrode, a small measuring volume around the sensor electrode is separated from the solution by a permeable membrane. Membrane-free sensors have been developed for miniaturized applications, wherein the sensor electrode is accommodated in a recess being in fluid connection with the solution (recessed electrode), or it is directly exposed to the solution (open-type electrode). Open-type electrodes are known from commercially available systems used in practice for sensing biomolecules. These electrodes generally suffer from disadvantages in terms of insufficient operation stability and limited biocompatibility. Sensing in tissue or blood results in the unintended coating of electrodes with cells or proteins. With a recessed electrode, operation stability can be improved and distortions of the current signal resulting from fluid convections can be minimized.

Conventionally, a recessed electrode can be manufactured using a coating and etching process as described by T.-S. Lim et al. in "Sensors and Actuators B" (vol. 141, 2009, p. 50 - 57). Briefly, a rod-shaped glass core is coated with a metallic electrode layer and an insulating layer. Subsequently, the glass core is cut and etched at one end so that an inner hollow space (recess) is formed which is enclosed by the electrode and insulating layers. This conventional method may have disadvantages in terms of complexity of the manufacturing process and reproducibility of the recess formation. As the recess shape and dimensions influence the measured current signals, the conventional recessed sensor electrode have a limited precision and reproducibility.

Another biosensor electrode is described in US 6 514 718 B1 for sensing glucose. An electrode wire carrying an insulating cladding is etched at one end such that the wire is removed and a cylindrical space is formed. Due to the application of the etching process, the manufacturing of the biosensor electrode has the same disadvantages as noted above. Furthermore, as the cylindrical space is completely filled with membrane and gel materials, the biosensor electrode is not a recessed electrode.

A further disadvantage of the conventional recessed electrodes is related to the fact that they are exclusively adapted for electrochemical sensing. Other measuring principles cannot be implemented with the conventional structures.

### Objective of the invention

The objective of the invention is to provide an improved sensor device, in particular for molecule sensing purposes, which is capable of avoiding disadvantages of conventional techniques. In particular, the objective is to provide a sensor device which enables molecule sensing with improved precision and/or reproducibility. Another objective of the invention is to provide an improved measuring apparatus including at least one sensor device. Furthermore, the objective of the invention is to provide an improved method of manufacturing a sensor device, in particular for molecule sensing purposes.

### Summary of the invention

The above objective is solved by a sensor device, a measuring apparatus and/or a manufacturing method comprising the features of the independent claims. Advantageous embodiments of the invention are defined in the dependent claims.

According to a first aspect of the invention, a sensor device, in particular being adapted for sensing of substances, including a sensor filament and a recessed sensor tip is provided. The sensor filament has a first end providing a sensing surface and a second end being connectable with a metering device. The recessed sensor tip forms an inner space (empty space, recess) enclosing the sensing surface. According to the invention, a first capillary (first hollow capillary) is provided, which includes the sensor filament. At a first capillary end of the first capillary, the sensor filament, i. e. the sensing surface thereof is exposed. Furthermore, according to the invention, a second capillary (second hollow capillary) is provided, which is fixedly connected to the first capillary end and which forms the recessed sensor tip. The sensor filament is exposed to the inner space of the second capillary.

The first and second capillaries are tube-shaped components, preferably with a circular cross-section, e. g. made of silica, glass or a solid plastic material. Both the first and second capillaries are made separately before being connected, and the first and second capillaries are connected via a connecting plane, e. g. by bonding, gluing or adhering. As an advantage of the invention, the second capillary is a pre-formed self-contained part of the sensor device, so that the recess can be provided with high precision in terms of size and shape. Limitations of conventional sensor electrodes resulting from irreproducible etching steps can be avoided.

The sensor device according to the invention has the particular advantage that it is capable of providing a robust, easily handle device, whereas many of the conventional techniques use fragile electrodes. Furthermore, the inventive sensor device can be used under practical conditions, in particular in human monitoring. Furthermore, using multiple filament tracks in the hollow fibers coupled with multiple recesses in such fibers there is the potential to have miniature electrochemical arrays for multiple measurements. According to a second aspect of the invention, a measuring apparatus, in particular being adapted for sensing of substances, is provided, which includes at least one sensor device according to the above first aspect of the invention, a reference device, and a metering device connectable with the sensor and reference devices. For multi-channel measurements, the measuring apparatus includes a plurality of sensor devices. The sensor devices can be fixedly connected as a multi-channel unit, which represents an independent aspect of the invention.

According to a third aspect of the invention, a method of manufacturing a sensor device, in particular the sensor device according to the above first aspect of the invention, is provided, which comprises a first step (A) of providing a first capillary including a sensor filament and having a first capillary end. A sensing surface of the sensor filament is exposed at the first capillary end. The sensing surface is surrounded by the end face (front side) of the first capillary. Furthermore, the manufacturing method comprises a second step (B) of providing a second capillary, which has an inner space (empty space). With a third step (C), first and the second capillaries are fixed to each other, so that the sensing surface is exposed to the inner space and a recessed sensor tip is formed. As advantages of the invention, the manufacturing method is essentially facilitated compared with conventional techniques, and the recess formation can be obtained with improved reproducibility. As the recess shape and dimensions are determined by the pre-formed second capillary, the manufacturing method yields in a recessed sensor electrode having improved precision and reproducibility.

With particularly advantageous applications of the invention, glucose can be sensed, which is central for diabetes monitoring in the home and in the hospital setting. Furthermore, lactate can be sensed, in particular for determining the severity of shock states, where there is not sufficient blood going to the peripheral tissues and organs. As a further example, hydrogen peroxide can be measured, which is generated in many tissues responses that involve inflammation as well as a number of biological events and intracellular pathways that have been linked to diseases. Finally, oxygen is preferably measured, which gives direct measure of the effectiveness of lung and cardiac function, including in shock, and is used frequently in cardiovascular and respiratory disorder monitoring.

According to a preferred embodiment of the invention, the sensor device is provided with an outer cladding, which extends over a connecting region of the first and second capillaries. Advantageously, the outer cladding provides an improved mechanical stability of the sensor device. For manufacturing this embodiment of the sensor device, above step (C) preferably includes a fixing of the outer cladding on an outer connecting region extending from the first and to the second capillary.

According to a particularly preferred embodiment of the invention, the outer cladding comprises a third capillary, which is arranged for covering the first capillary an the first capillary end thereof and at least a portion of the second capillary. The third capillary is a tube-shaped component having an inner diameter adapted to the outer diameter of at least one of the first and second capillaries. Preferably, the third capillary has a circular cross-section, and it is e. g. made of glass or a solid plastic material. In accordance with this embodiment, the above step (C) preferably includes a fixing of the third capillary on an outer side of the first capillary end and the second capillary.

According to a further advantageous embodiment of the invention, the second capillary is glued to the first capillary end. Accordingly, above step (C) preferably includes connecting the first and second capillaries by applying a glue to the first capillary end of the first capillary. Using an UV setting or thermosetting glue is particularly preferred as these types of glues facilitate a quick connecting step, so that a risk of maladjustment of the capillaries is minimized.

According to a further variant of the invention, the first and second capillaries may have equal outer diameters. With this embodiment, attachment of the outer cladding, in particular the third capillary is facilitated. Additionally or alternatively, the second capillary may have an inner diameter which is equal or smaller than a diameter of the sensor filament. Thus, the second capillary forms a frame defining a size of the exposed area of the sensor filament. Advantageously, this further improves the precision of the sensor device.

The sensor filament is a component having a longitudinal extension, which is arranged in the hollow inner space of the first capillary. The particular structure of the sensor filament depends on the sensing mechanism used for sensing the substance. According to a preferred example, the sensor filament is an electrode filament, i. e. an electrically conductive wire, in particular made of gold. According to further preferred examples, the sensor filament may be an optical filament, i. e. an optical waveguide like an optical fibre, in particular made of silica or soft glass, an impedimetric transducer, and an electrochemical transducer. In a multi-channel unit, multiple sensor filaments are provided, which could have identical types and may comprise e. g. electrode filaments, or they could have different types, wherein e. g. at least one electrode filament can be combined with at least one optical filament.

The first capillary accommodates the sensor filament, so that a protection of the sensor filament and a connection of the sensing surface via the sensor filament to the metering device is obtained. To this end, a maximum outer diameter of the sensor filament is smaller than or equal to the inner diameter of the first capillary. If a gap between the sensor filament and the first capillary end is formed, this gap is preferably closed with a sealing substance, in particular a glue.

The recessed sensor tip can be designed in dependency on the sensing mechanism used for sensing the substance and the conditions of the substance. As an example, the recessed sensor tip may open towards an environment of the sensor device, so that advantages in terms of a short response time of the sensor device are obtained. As an alternative example, the recessed sensor tip may be covered by a membrane separating the inner space from an environment of the sensor device. In this case, the sensing surface may be protected against unintended alterations, e. g. protein layers or adsorbing cells included in the tissue or solution to be measured. Additionally or alternatively, the sensing surface of the filament may be itself be covered by a membrane, separating it from the inner space. In this case the sensing surface is protected from unintended alterations inside the inner space of a recess tip that opens towards an environment of the sensor device. Additionally or alternatively, the recessed sensor tip may be provided with a predetermined longitudinal length selected in dependency on the environment of the sensor device during measurement. In the latter case, the method of the invention preferably includes a sub-step of adjusting an axial length of the recessed sensor tip by cutting the second capillary.

According to a particularly advantageous embodiment of the invention, at least one of the first and second capillaries comprises a photonic crystal fibre (PCF). With this embodiment, advantages result from the high precision of shaping an dimensioning of PCF.

According to a further preferred feature of the invention, antibody molecules can be linked to the sensing surface. In this case, it would be possible to extend the measurement to any target for which an antibody molecule can be generated, like e.g. therapeutic drugs and drugs of abuse for which commercial antibodies are already available.

Further advantages of the invention may be obtained the first and second capillaries are connected along polished capillary end. Accordingly, the method of the invention preferably comprises at least one of polishing the first capillary end such that is aligned with the sensing surface of the sensor filament and polishing the second capillary at an end to be connected with the first capillary.

### Brief description of the drawings

Further details and advantages of the invention are described in the following with reference to the attached drawings, which show in:
- Figure 1:: a schematic cross-sectional view of a preferred embodiment of a sensor device according to the invention;
- Figure 2:: a schematic view of a preferred embodiment of a measuring apparatus according to the invention;
- Figures 3 to 5:: a schematic illustrations of steps of a preferred embodiment of a manufacturing method according to the invention; and
- Figure 6:: an exemplary view of a preferred embodiment of a multi-channel unit according to the invention.

### Description of the preferred embodiments

Preferred embodiments of the invention are described in the following with exemplary reference to electrochemical sensing of substances. The implementation of the invention is not restricted to electrochemical sensing, but rather can be obtained in an analogue manner e. g. with optical sensing. The invention can be applied in combination with conventional substance sensing techniques. Known details e. g. of electrochemical sensing of substances like e. g. calibration and measuring procedures, selection of electrode materials, operation of a metering device and the like, are not described here.

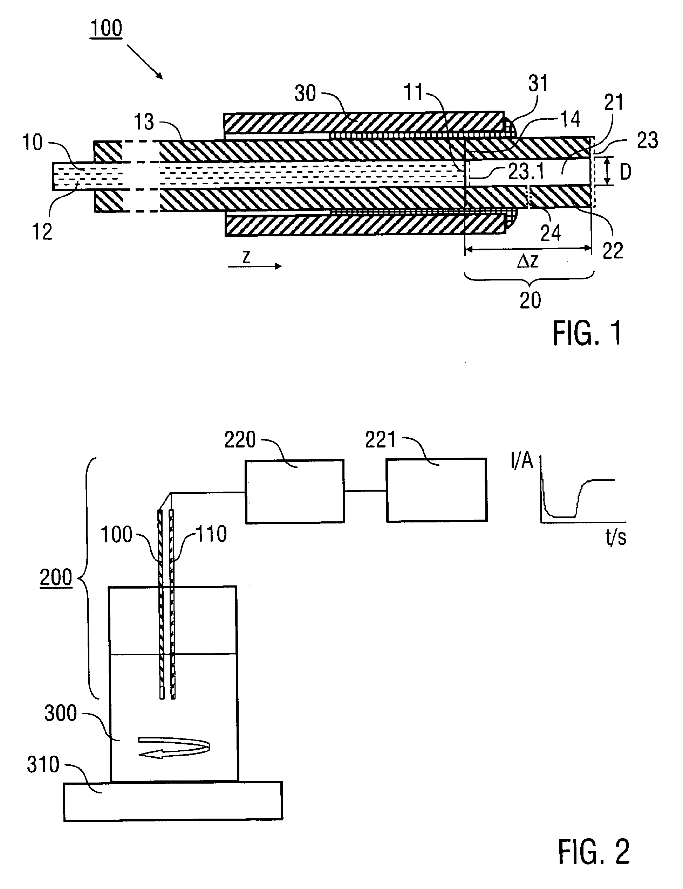

Figure 1 shows a first embodiment of a sensor device 100 with an enlarged schematic cross-sectional view. The sensor device 100 extends in a longitudinal direction (axial direction, z-direction) from a recessed sensor end to a metering device.

For clarity reasons, Figure 1 does not show the whole length of the sensor device 100 but only the recessed sensor end thereof. Depending on the application of the invention, the length of the sensor device 100 may be selected to be at least of 0,5 cm, in particular at least 1 cm, e. g. 5 cm or more, like 20 cm or longer. Depending on the materials and dimensions, the sensor device 100 may be rigid or bendable. The bendable design has advantages for a combination of the sensor device with a flexible catheter, e. g. for medical investigations.

The sensor device 100 comprises a sensor filament 10 accommodated in a first capillary 13, a recessed sensor tip 20 made by a piece of a second capillary 22 which is fixed to the first capillary 13, and an outer cladding 30 covering a connecting region of the first and second capillaries 13, 22. The capillaries preferably have cylindrical geometry as shown. Alternatively, the capillaries may have another cross-sectional shape, like e. g. an elliptic shape.

The sensor filament 10 is a gold or platinum wire with a diameter selected in the range of 20 µm to 80 µm. The wire has a first end providing a sensing surface 11 and a second end 12 being connectable with a metering device (see Figure 2, reference numeral 220). The sensing surface 11 is aligned with the first capillary end 14, e. g. as a result of a common polishing of the first capillary end 14 with the inner wire.

Both of the first capillary 13 and the second capillary 22 are photonic crystal fibres being made of e. g. silica or soft glass, and each having an inner diameter D of at least 50 µm and at most 150 µm. The second capillary 22 has an empty inner space 21, which is delimitated by the sensing surface 11 and the inner wall of the second capillary 22. The axial length Δz of the second capillary 22 is at least 50 µm and at most 10 mm. The outer diameters of each of the first and second capillaries 13, 22 is at least 80 µm and at most 1000 µm. Both capillaries may have equal or different diameters.

The outer cladding 30 comprises a third capillary, like e. g. a glass capillary with an inner diameter being matched to the maximum outer diameter of the first and second capillaries 13, 22. The outer cladding 30 is fixed to the first and second capillaries 13, 22 with a glue 31. The axial length of the third capillary 30 is at least 100 µm and at most 10 mm.

Additionally, Figure 1 shows further optional features of the inventive sensor device 100. According to a variant of the invention, a membrane 23 can be provided covering the open end of the second capillary 22 (shown with dashed line). The membrane 23 is made of e. g. polycarbonate, cellulose acetate or Nafion, optionally incorporating connected pores and having a thickness of e. g. 20 µm. Furthermore, a membrane 23.1, e. g. made from the same membrane material, can be provided directly covering the sensing surface of the filament 11. Furthermore, at least one side through-hole 24 can be provided in a side wall of the second capillary 22 (shown with dotted line). The through-hole 24 has a diameter of e. g. 50 µm, it is formed e. g. by drilling or local etching. A further membrane can be provided covering the through-hole 24.

Figure 2 schematically illustrates a preferred embodiment of a measuring apparatus 200 according to the invention. The measuring apparatus 200 comprises a sensor device 100 according to the invention, in particular as described above with reference to Figure 1, a reference device 110, and a metering device 220 which is connected with the sensor and reference devices 100, 110. With the preferred application of electrochemical sensing, the reference device 110 comprises a reference electrode, and all standard potentiostats can be used as the metering device 220, including e. g. miniaturized units as commercially used for glucose sensing. The metering device 220 can be connected with a control and/or evaluation device 221, embodied e. g. by a microprocessor. As an example, the reference electrode is a standard component, like a wire structure made of Ag/AgCl, which is used to provide a stable reference potential against which the working electrode of the recessed electrode is polarized. As a further example, the reference electrode is a metal tube, e. g. a stainless steel tubing, wherein in a particular embodiment the inventive recessed tip electrode can be held within the lumen of the tube to give a single monolithic structure for ease of application.

As illustrated, the measuring apparatus 200 can be used for sensing a substance under investigation in a solution arranged in a container 300, e. g. a cuvette, beaker or flask positioned on a conditioning device 310, e. g. a heating and/or stirring device. Alternatively, sensing can be conducted with biological subjects, e. g. in vivo in living tissue or in a vessel, like a blood vessel.

Substances to be sensed with the inventive sensor device 100, in particular with the measuring apparatus 200 according to Figure 2 may comprise at least one of the molecules or compounds mentioned in the following. Depending on the electrochemical reaction at the sensing surface 11, the substance can be sensed directly, or a reaction product can be measured, which creates an electrochemical sensor signal (current signal) being a direct measure for the substance to be detected.

Firstly, there is a group of oxidase enzymes that can degrade complex organic molecules. Metabolites that can be degraded by oxidase enzymes include glucose, lactate, pyruvate, cholesterol and amino acids. These generate readily measurable hydrogen peroxide. Furthermore, hydrogen peroxide or oxygen can be sensed as such. Furthermore, there is the possibility to use dehydrogenase enzymes if coupled with a second (indicator) reaction. With this regard, the recess structure provides a particular advantage in terms of housing such an indicator reaction. Alternatively, the enzyme molecules can be linked to the sensing surface or used as a chemically or physically immobilised layer over the sensing surface. Furthermore, antibody molecules can be linked to the sensing surface 11 so that target molecules coupling with the antibody can be sensed.

If instead of an electrochemical sensing, an optical sensing is provided and an optical fibre is used as the sensor filament, than all target molecules can be sensed, which can be monitored with optical measurements in particular with optical fibres. These include in particular the above components, but more easily it is possible to measure pH values and CO₂.

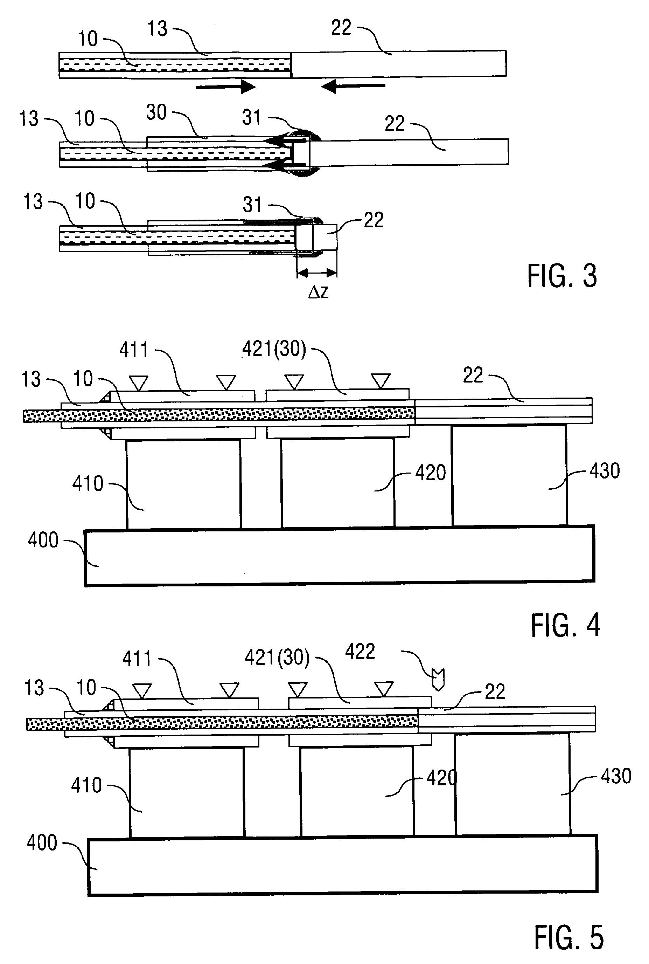

Features of preferred embodiments of the method of manufacturing the sensor device 100 are described in the following with reference to Figures 3 to 5. Figure 3 gives a general overview of the method steps, while further details are shown in Figures 4 and 5.

According to Figure 3 (upper image), the inventive method of manufacturing the sensor device comprises a step A of providing the first capillary 13 filled with the sensor filament and a step B of providing the second capillary 22 in mutual contact to each other. With step C, the second capillary 22 is connected to the end of the first capillary 13 so that the end of the sensor filament 10 is exposed to the inner space of the second capillary 22.

According to the centre image of Figure 3, an outer cladding 30 is provided for connecting the first and second capillaries. The outer cladding 30 comprises the third capillary, which is glued to both of the first and second capillaries 13, 22. Depending on the pre-forming of the second capillary 22, a final step of cutting the length of the recess can be provided as shown in the bottom image of Figure 3. With further details, the procedure includes the following steps.

Step A of providing the first capillary 13 includes a step of preparation of the capillary. A gold filled capillary is provided e. g. by vacuum based sucking of liquefied (molten) gold into the capillary or by introducing a gold wire. The gold filled capillary polished at the free end thereof using 5 µm, 3 µm and 1 µm Aluminium Oxide film in consecutive order. The polished tip is placed into medium viscosity UV glue drop with the aim to cover with the glue the possible air gap between the capillary wall (silica) and the gold wire inside the capillary 13. After applying UV light to make the glue solid, the first capillary 13 is polished sequentially again using 5 µm, 3 µm and 1 µm Aluminium Oxide film. Than the polished gold tip is cleaned using acetone ultrasound bath for 2 min, washed in the de-ionized water and air dried at room temperature for 1 hour.

The second capillary 22 (empty capillary/PCF) is prepared by removing a polymer cladding of approximately 10 cm of the PCF and by cleaving one tip of the PCF, which helps to achieve a perfect flat tip surface. Preferably, the outer diameter of the cleaned PCF should be equal to the one of the first capillary 13. The inner diameter of the second capillary 22 should be equal or smaller than the diameter of the sensor filament 13 (gold wire diameter).

Subsequently, with step C, the first and second capillaries 13, 22 are connected to each other. To this end, an optical fibre positioning system 400 is used, which comprises three fibre holders 410, 420 and 430. Optical fibre positioning systems 400 are conventionally known from fibre optics and techniques for coupling optical waveguides. Connecting the first and second capillaries 13, 22 is obtained using a first auxiliary capillary 411 and a second auxiliary capillary 421, which subsequently provides the third capillary 30. The first and second auxiliary capillaries 411, 421 and the third capillary 22 are placed on the first to third fibre holders 410 to 430, resp..

The first capillary 13 is inserted into the two larger diameters empty auxiliary capillaries 411, 421. The first auxiliary capillary 411 is fixed from one side to the first capillary 13 using an UV glue 412. The second auxiliary capillary 421 is left unfixed to allow its movement over a connecting region between the first and second capillaries 13, 22. Subsequently, the optical fibre positioning system 400 optionally in combination with a magnifying glass are used to achieve the connection between the ends of the first and second capillaries 13, 22.

Subsequently, the first and second capillaries are fixed to each other as shown in Figure 5. The second fibre holder 420 is not clamped to the optical fibre positioning system 400 in order to allow a movement and the correct adjustment of the second auxiliary capillary 421 (30) over the connecting region. After the overlapping at the connecting region is achieved, an UV glue drop 422 is applied as shown in Figure 5.

Since the glue is in fluid state initially, the air gap between the second auxiliary capillary 421 (30), the first capillary 13 and the second capillary 22 is filled by the UV glue due to the capillary effect. Thus, the structures are joined to each other. Subsequently, UV light is applied within 1 min to produce a rigid fixation.

Finally, the longitudinal length of the recess is set by cleaving the second capillary 22 using a ceramic tool. Optionally, a further improvement can be achieved by applying a platinization of the gold tip, which acts as the sensing surface.

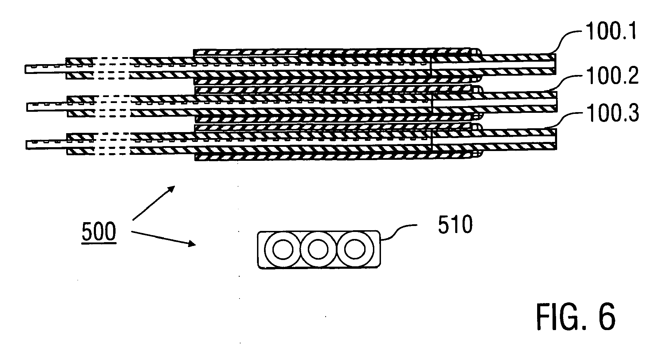

For multi-channel applications, a plurality (of at least 2) sensor devices 100 according to the invention can be combined as schematically shown in Figure 6. In the illustrated example, the embodiment of the multi-channel unit 500 comprises three sensor devices 100.1, 100.2 and 100.3 each be structured like the sensor device of Figure 1 and manufactured as described above with reference to Figures 3 to 5. The sensor devices 100.1, 100.2 and 100.3 are connected to each other along lateral sides thereof and/or enclosed by a casing 510 as shown with the cross-sectional view in the lower portion of Figure 6.

With the embodiment of Figure 6, the sensor comprises multiple sensing surfaces and multiple recesses to create electrode arrays. There would also be possible different enzyme and other membranes to modify the performance of the device. It would be possible to create access to the recess from a side of the recess wall for the analyte, and a space between the electrode and the walls could be provided to allow the flow of fluid down the sides to the recess. Also the walls of the recess rather than the surface of the working electrode could retain the enzyme for a different type of performance.

The features of the invention disclosed in the above description, the figures and the claims can be equally significant for realising the invention in its different embodiments, either individually or in combination.

## Claims

1. Sensor device (100), in particular being adapted for sensing of substances, which comprises
- a sensor filament (10), which has a first end providing an exposed sensing surface (11) and a second end (12) being connectable with a metering device (220), and
- a recessed sensor tip (20), which has an inner space (21) to which the sensing surface (11) is exposed,
**characterized by**
- a first capillary (13) including the sensor filament (10) and having a first capillary end (14), where the sensing surface (11) is exposed to the inner space (21) of the recessed sensor tip (20), and
- a second capillary (22) being fixedly connected to the first capillary end (14) and providing the recessed sensor tip (20).

2. Sensor device according to claim 1, comprising
- an outer cladding (30) covering a connecting region of the first and second capillaries (13, 22).

3. Sensor device according to claim 2, comprising
- the outer cladding (30) comprises a third capillary extending from the first capillary end (14) to the second capillary (22).

4. Sensor device according to one of the foregoing claims, wherein
- the second capillary (22) is fixedly connected to the first capillary end (14) by a glue (31).

5. Sensor device according to one of the foregoing claims, wherein
- the first and second capillaries (13, 22) have equal outer diameters, and/or
- the second capillary (22) has an inner diameter being equal or smaller than a diameter of the sensor filament (10).

6. Sensor device according to one of the foregoing claims, wherein
- a gap between the sensor filament (10) and the first capillary end (14) is closed with a sealing substance, in particular a glue.

7. Sensor device according to one of the foregoing claims, wherein
- the recessed sensor tip (20) opens towards an environment of the sensor device (100), or
- the recessed sensor tip (20) is covered by a membrane (23) separating the inner space (21) from an environment of the sensor device (100), and/or the surface (11) of the sensor filament (10) is covered by a membrane (23.1) to separate it from the inner space of the recess tip (20).

8. Sensor device according to one of the foregoing claims, comprising at least one of the features:
- at least one of the first and second capillaries (13, 22) is made of silica,
- at least one of the first and second capillaries (13, 22) comprises a photonic crystal fibre (PCF),
- the sensor filament (10) is an electrode filament, in particular made of gold, platinum or gold with a platinum surface,
- the sensor filament (10) is an optical filament, in particular made of silica or soft glass,
- the sensor filament (10) is an impedimetric transducer,
- the sensor filament (10) is an electrochemical transducer,
- at least one type of enzyme or antibody molecules is linked to at least one of the sensing surface (11) and inner walls of the inner space (21), and
- the recessed sensor tip (20) has a side through-hole.

9. Measuring apparatus (200), in particular being adapted for sensing of substances, comprising:
- at least one sensor device (100) according to one of the foregoing claims,
- a reference device (110), and
- a metering device (220) connectable with the sensor and reference devices (100, 110).

10. Method of manufacturing a sensor device (100), in particular the sensor device (100) according to one of the claims 1 to 8, comprising the steps of:
(A) providing a first capillary (13) including a sensor filament (10) and having a first capillary end (14), where a sensing surface (11) of the sensor filament (10) is exposed,
(B) providing a second capillary (22) having an inner space (21), and
(C) fixedly connecting the second capillary (22) to the first capillary end (14) of the first capillary (13), so that the sensing surface (11) is exposed to the inner space (21) and a recessed sensor tip (20) is formed.

11. Method according to claim 10, wherein step (C) comprises:
(C1) fixing an outer cladding (30) covering a connecting region of the first and second capillaries (13, 22).

12. Method according to claim 11, wherein:
- the outer cladding (30) comprises a third capillary extending from the first capillary end (14) to the second capillary (22).

13. Method according to one of the claims 10 to 12, wherein step (C) comprises:
(C2) connecting the second capillary (22) to the first capillary end (14) of the first capillary (13) using a glue (31), in particular an UV setting or thermosetting glue.

14. Method according to one of the claims 10 to 13, wherein step (C) further comprises:
(C3) adjusting an axial length of the recessed sensor tip (20) by cutting the second capillary (22).

15. Method according to one of the claims 11 to 14, wherein steps (A) and (B) comprise at least one of:
- polishing the first capillary end (14) such that is aligned with the sensing surface (11) of the sensor filament (10),
- filling a gap between the sensor filament (10) and the first capillary end (14) with a sealing substance, in particular a glue, and
- polishing the second capillary (22) at an end to be connected with the first capillary (13).
